(19) Europäisches Patentamt
European Patent Office
Office européen des brevets

(11) Veröffentlichungsnummer: **0 624 614 A1**

# (12) EUROPÄISCHE PATENTANMELDUNG

(21) Anmeldenummer: **94106666.4**

(22) Anmeldetag: **28.04.94**

(51) Int. Cl.5: **C08G 63/91**, C08G 64/42, C07C 231/02, C07C 269/04

(30) Priorität: **11.05.93 DE 4315662**

(43) Veröffentlichungstag der Anmeldung: **17.11.94 Patentblatt 94/46**

(84) Benannte Vertragsstaaten: **AT BE CH DE ES FR GB IT LI NL**

(71) Anmelder: **BAYER AG**

**D-51368 Leverkusen (DE)**

(72) Erfinder: **Scholl, Hans-Joachim, Dr.**
**Am Feldrain 5**
**D-51061 Köln (DE)**
Erfinder: **Grögler, Gerhard, Dr.**
**von-Diergardt-Strasse 48**
**D-51375 Leverkusen (DE)**

## (54) Verfahren zur Umsetzung von Ester- oder Carbonatgruppen zu Amid oder Urethangruppen.

(57) Es wird ein Verfahren zur Herstellung von aliphatisch gebundene Amid- oder Urethangruppen enthaltenden Verbindungen beschrieben, bei dem aliphatisch gebundene Ester- oder Carbonatgruppen enthaltende Verbindungen mit mindestens einer aliphatisch gebundene Amingruppe enthaltenden Verbindungen in Gegenwart von katalytisch wirksamen Mengen an Alkalialkylaten und Alkylformiaten umgesetzt werden.

EP 0 624 614 A1

Die vorliegende Erfindung betrifft ein Verfahren zur Herstellung von aliphatisch gebundene Amid- oder Urethangruppen enthaltenden Verbindungen aus aliphatisch gebundene Ester- oder Carbonatgruppen enthaltenden Verbindungen, das dadurch gekennzeichnet ist, daß aliphatisch gebundene Ester- oder Carbonatgruppen enthaltende Verbindungen mit mindestens eine aliphatisch gebundene Amingruppe enthaltenden Verbindungen in Gegenwart von katalytisch wirksamen Mengen an Alkalialkylaten und Alkylformiaten umgesetzt werden.

Die erfindungsgemäße Reaktion kann für Carbonatgruppen enthaltende Verbindungen allgemein durch das nachfolgende Reaktionsschema beschrieben werden:

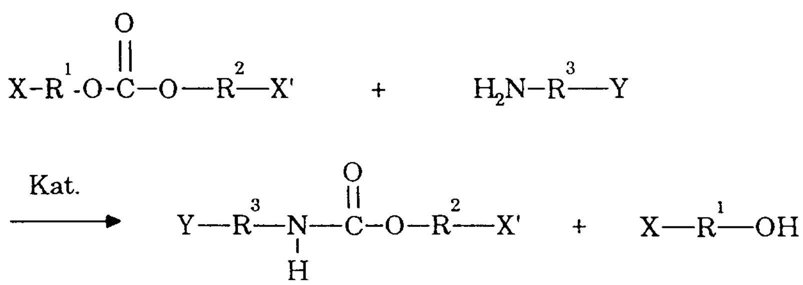


Dabei bedeuten:

- X, X' = -H, OH oder -OR (gleich oder verschieden)
- Y = -H, -OH, $-NH_2$, -OR
- R = Alkyl-, Cycloalkyl- oder Aralkyl-Rest
- $R^1$, $R^2$ = gleiche oder verschiedene organische Reste, die gegebenenfalls weitere Carbonatgruppen aufweisen können,
- $R^3$ = aliphatischer oder cycloaliphatischer Rest.

Die Durchführung der Reaktion erfolgt derart, daß die Ester- oder Carbonatgruppen enthaltende Verbindung und die Amingruppen enthaltende Verbindung miteinander vermischt werden und entweder anschließend die Katalysatorsubstanzen zugemischt werden oder aber die Katalysatorsubstanzen bereits vorab einer der beiden reagierenden Verbindungen zugemischt wurden.

An die Reaktionspartner ist lediglich die Bedingung zu knüpfen, daß zumindest einer der Reaktionspartner bei Raumtemperatur oder bei leicht erhöhter Temperatur flüssig ist und der andere Reaktionspartner entweder ebenfalls flüssig ist oder in dem flüssigen Reaktionspartner löslich ist.

Von erfindungswesentlicher Bedeutung ist die gleichzeitige Verwendung katalytischer Mengen an Alkalialkylaten und Alkylformiaten. Als Alkalialkylate werden bevorzugt Methylate und/oder Ethylate des Lithiums, Natriums oder Kaliums, besonders bevorzugt Natriummethylat, eingesetzt.

Als Alkylformiate werden bevorzugt Methylformiat und/oder Ethylformiat, besonders bevorzugt Methylformiat, eingesetzt.

Die beiden Katalysatorsubstanzen werden bevorzugt in einem molaren Verhältnis von 1:3 bis 3:1 eingesetzt.

Im Falle des Einsatzes von Methylformiat liegen katalytisch wirksame Mengen im allgemeinen bei 0,01 bis 0,08 Mol, vorzugsweise 0,01 bis 0,05 Mol pro Mol Aminogruppen der aliphatische Aminogruppen enthaltenden Verbindung.

Im Falle des Natriummethylats liegen katalytisch wirksame Mengen in der Regel bei 0,01 bis 0,1 Mol, vorzugsweise 0,01 bis 0,08 Mol, pro Mol Aminogruppe der aliphatisch gebundene Aminogruppen enthaltenden Verbindung.

Die erfindungsgemäße Reaktion wird im allgemeinen bei Normaldruck und bei Temperaturen zwischen 20 bis 120 °C, vorzugsweise 30 bis 80 °C durchgeführt.

Gegenstand der vorliegenden Erfindung ist auch das Katalysatorsystem zur Reaktion von aliphatisch gebundenen Ester- bzw. Carbonatgruppen mit aliphatisch gebundenen Amingruppen unter Erzeugung von Amid- bzw. Urethangruppen, das aus den Komponenten Alkalialkylate und Alkylformiate besteht.

Bevorzugt wird das erfindungsgemäße Katalysatorsystem zur Herstellung eines Polyolgemisches mit freien Amino-Endgruppen durch Umsetzung eines Hydroxylgruppen enthaltenden, aliphatischen Polycarbonats oder Polyesters mit einem aliphatischen Polyamin eingesetzt.

Die erfindungsgemäß bevorzugte Reaktion kann für das Beispiel Polycarbonat durch das folgende Reaktionsschema verdeutlicht werden:

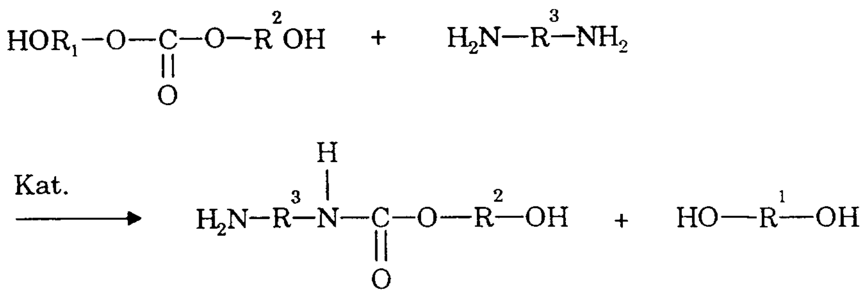


dabei stehen $R^1$ und $R^2$ für Carbonatgruppen enthaltende Wiederholungseinheiten des Polycarbonats und $R^3$ stellt einen beliebigen aliphatischen oder cycloaliphatischen Rest dar.

Erfindungsgemäß kann die Reaktion des Polyamins mit dem Polycarbonat oder Polyester durch Säurezusatz gestoppt werden.

Zur Herstellung der erfindungsgemäß bevorzugten Polyolgemische mit freien Amino-Endgruppen wird die Reaktion gestoppt, nachdem mindestens eine Aminogruppe des Polyamins zur Amid- oder Urethangruppe umgesetzt ist.

Bevorzugt werden zum Abstoppen der Reaktion anorganische oder organische Säuren mit einem pKa-Wert von unterhalb 3 eingesetzt. Geeignete Säuren zum Stoppen der Reaktion sind Methansulfonsäure, Schwefelsäure oder Phosphorsäure, die vorzugsweise in äquivalenten Mengen zum Alkalikatalysator eingesetzt werden.

Der Fortgang und der Zeitpunkt der Beendigung der Reaktion lassen sich einfach durch IR-Analyse, nämlich Abnahme einer Bande bei 1590 $cm^{-1}$, die dem freien (Poly-)amin im Gemisch zugeordnet wird, der Brechungsindexzunahme oder der Bestimmung der Abnahme des "Basen-N", d.h. prozentuale Abnahme des Stickstoffs der freien Aminogruppen ermitteln.

Bevorzugte Ausgangsverbindungen für die erfindungsgemäß bevorzugte Reaktion sind einerseits aliphatische Polycarbonate mit Hydroxylgruppen, wie sie als Diolkomponente zur Synthese von Polyurethankunststoffen herangezogen werden, siehe z.B. Encyclopedia of Polymer Science and Enginering Vol. 11, Seite 648 bis 650, New York 1988 oder aliphatische Polyester mit Hydroxylg ruppen, siehe z.B. Kunststoff-Handbuch "Polyurethane", Band 7, herausgegeben von G. Oertel, S. 54-62, München 1983, sowie andererseits (Poly-)amine mit mindestens zwei aliphatisch gebundenen primären Aminogruppen.

Die erfindungsgemäß bevorzugt erhaltenen Polyolgemische mit freien Aminoendgruppen weisen vorzugsweise OH-Zahlen von 100 bis 140 bei einem Aminstickstoffgehalt (berechnet als N, Atomgewicht 14) von 0,4 bis 1,0 Gew.-% auf. Solche Polyolgemische mit freien Amino-Endgruppen stellen wertvolle Ausgangsstoffe für die Herstellung von Polyurethanen nach dem Polyisocyanat-Polyadditionsverfahren dar, insbesondere sind sie zum Einsatz als Kettenverlängerungsmittel geeignet.

## Ausführungsbeispiele

A) Herstellung der erfindungsgemäßen Polyolgemische

Ausgangsprodukte

Polycarbonat 1: Polycarbonat auf Basis Hexandiol-1,6/Caprolacton (1:1-molar), Diphenylcarbonat
OHZ: 57,6, MG: 2000
Polycarbonat 2: Polycarbonat auf Basis Hexandiol-1,6, Diphenylcarbonat
OHZ: 56, MG: 2000
Polyester 1: Polyester auf Basis Adipinsäure/Hexandiol-1,6/Neopentylglycol (1:0,73:0,49; molar),
OHZ: 66
Amin 1: 2-Methylpentamethylendiamin-1,5 ("DYTEK-A" der Fa. DuPont)
Amin 2: Jeffamin T403 (Triamin durch Aminierung der endständigen OH-Gruppen eines Polyethers auf Basis Trimethylpropan/Propylenoxid;
MG: 440; Basen-N: 9,5 %; Fa. Texaco).
Amin 3: 5-Amino-1-aminomethyl-1,3,3-trimethylcyclohexan
Amin 4: 1,6-Diaminohexan

**Beispiel 1** (erfindungsgemäßes Verfahren)

1200 g entwässertes Polycarbonat 1 werden mit 69,7 g (0,6 Mol) Amin 1 bei 40 °C vermischt. Unter Rühren werden nachfolgend bei 40 °C a) 3 g Methylformiat und b) 2,2 g einer 30 %igen Natriummethylatlösung (in Methanol) zugetropft. Nach 4 Stunden Rührzeit bei 40 °C wird mit 1,2 g Methansulfonsäure abgestoppt und im Vakuum entgast. Man erhält ein klares Polyolgemisch mit den Daten:
OHZ: 112 Viskosität (23 °C): 5000mPa.s
Basen-N: 0,7 %

$$n_D^{23^\circ C}: 1.4704$$

Im Verlauf der Reaktion nahm die IR-Bande bei ~1590 $cm^{-1}$ ab (Aminbande) und entsprechend eine Bande bei ~1510 $cm^{-1}$ (Urethanbande) zu.

**Beispiel 2** (Vergleich)

Beispiel 1 wird exakt ohne Mitverwendung von Methylformiat wiederholt. Es tritt keine erfindungsgemäße Reaktion ein. Die Ausgangswerte "Basen-N" und "Brechungsindex" bleiben unverändert. Die IR-Bande bei ~1590 $cm^{-1}$ von gleichbleibender Intensität. Selbst ein nachfolgendes kurzes Aufheizen bis 100 °C bewirkt keine Änderungen.

**Beispiel 3** (Vergleich)

Beispiel 1 wird exakt ohne Mitverwendung von Methylformiat und Natriummethylat wiederholt. Es tritt keine erfindungsgemäße Reaktion ein. Das Gemisch bleibt unverändert.

**Beispiel 4** (erfindungsgemäßes Verfahren) 1200 g entwässertes Polycarbonat 1 werden mit 176 g (0,4 Mol) Amin 2 bei 50 °C vermischt. Unter Rühren werden 3 gMethylformiat und 3,9 g einer 30 %igen Natriummethylatlösung zugetropft. Nach 3 Stunden Rührzeit bei 60 °C werden weitere 1,0 g einer 30 %igen Natriummethylatlösung hinzugetropft. Nach insgesamt 7 Stunden Rührzeit bei 60 °C wird mit 2,7 g Methansulfonsäure abgestoppt und im Vakuum entgast. Man erhält ein klares Polyolgemisch mit den Daten:
OHZ: 106 Viskosität (23 °C): 6500 mPa.s
Basen-N: 0,8 %

$$n_D^{23^\circ C} : 1.4692$$

**Beispiel 5** (erfindungsgemäßes Verfahren)

Beispiel 1 wird exakt wiederholt mit der Ausnahme, daß anstatt Amin 1 69,7 g (0,6 Mol) Amin 4 verwendet werden und insgesamt 4 g Natriummethylatlösung zum Einsatz kommen. Man erhält ein Polyolgemisch mit den Daten:
OHZ: 114 Viskosität (23 °C): 4500 mPa.s
Basen-N: 0,63 %

$$n_D^{23^\circ C} : 1.4706$$

**Beispiel 6** (erfindungsgemäßes Verfahren)

12000 g entwässertes Polycarbonat 1 werden mit 102 g (0,6 Mol) Amin 3 bei 40 °C vermischt. Unter Rüh ren werden nachfolgend 3 g Methylformiat und portionsweise insgesamt 4 g Natriummethylatlösung zugetropft. Nach 4 1/2 Stunden Rührzeit bei 40 °C wird mit 2,1 g Methansulfonsäure abgestoppt und im Vakuum entgast. Man erhält ein klares Polyolgemisch mit den Daten:
OHZ: 112 Viskosität (23 °C): 7000 mPa.s

Basen-N: 0,66 %

$$n_D^{23^\circ C} : 1.4730$$

**Beispiel 7**

400 g entwässertes Polycarbonat 2 werden mit 58,7 g (0,13 Mol) Amin 2 bei 60 °C vermischt. Unter Rühren werden 2 g Methylformiat und insgesamt 3 g Natriummethylatlösung (30 % in Methanol) zugetropft. Nach 6 Stunden Rührzeit bei 60 °C wird mit 1,6 g Methansulfonsäure abgestoppt und im Vakuum entgast. Man erhält ein Polyolgemisch, das beim Abkühlen wachsartig erstarrt, mit den Daten:
OHZ: 104
Basen-N: 0,8 %

**Beispiel 8**

1200 g entwässertes Polycarbonat 2 werden mit 93 g (0,8 Mol) Amin 1 bei 60 °C vermischt. Unter Rühren werden 3 g Methylformiat und insgesamt 6 g Natriummethylatlösung (30 % in Methanol) zugetropft. Nach 7 Stunden Rührzeit bei 60 °C wird mit 3,3 g Phosphorsäure abgestoppt und im Vakuum entgast. Man erhzält ein Polyolgemisch, das beim Abkühlen wachsartig erstarrt, mit den Daten:
OHZ: 125
Basen-N: 0,9 %

**Beispiel 9**

1.050 g entwässerter Polyester 1 wird mit 70 g (0,6 Mol) Amin 1 bei 60 °C vermischt. Unter Rühren läßt man 3 g Methylformiat und anschließend 14 g einer 30%igen Natriummethylatlösung (in Methanol) zutropfen. Nach 24 h Rührzeit bei 80 °C wird mit 7,5 g Methansulfonsäure abgestoppt und im Vakuum entgast. Man erhält ein Polyolgemisch, das bei Raumtemperatur wachsartig wird, aber fließfähig bleibt, mit den Daten:
OHZ: 137
Basen-N: 0,7 %

**Beispiel 10**

1.050 g entwässerter Polyester 1 wird bei 80 °C mit einer Mischung aus 102 g (0,6 Mol) Amin 3 und 5 g Methylformiat verrührt. Unter Rühren läßt man nachfolgend 16 g einer 30%igen Natriummethylatlösung (in Methanol) zutropfen. Nach 20 Stunden Rührzeit bei 120 °C wird mit 8,5 g Methansulfonsäure abgestoppt und im Vakuum entgast. Man erhält ein fließfähiges, wachsartiges Polyolgemisch mit den Daten:
OHZ: 132
Basen-N: 0,7 %

B) Verwendungsbeispiele

Herstellung von Polyurethankunststoffen

**Beispiel 11**

100 g eines Polyolgemisches gemäß Beispiel 1 werden mit 26,2 g fein gemahlenem 1,5-Diisocyanatonaphthalin (NDI, Fp. 127 °, Teilchengröße: 20 bis 40 µm) vermischt. Dieses Gemisch ist bei Raumtemperatur lagerstabil, d.h. es erfolgt keine Reaktion des Isocyanates mit den Reaktivgruppen. Nach kurzem Entgasen im Waserstrahlvakuum wird der flüssige Ansatz in eine Form gegossen, die dann auf 120 bis 140 °C erhitzt wird. Es tritt rasch Verfestigung ein und man erhält einen transparenten Polyurethan-Kunststoff mit hoher Elastizität und einer Shore Härte A (DIN 53 505) von 90.

**Beispiel 12**

Werden in obigem Beispiel die 26,2 g NDI durch 50,9 g 4,4'-Dimethyl-3,3'-diaminodiphenylharnstoff (hergestellt aus 2 Mol 2,4-Diisocyanatotoluol und 1 Mol Wasser in Aceton) ersetzt, so erhält man wie in Beispiel 1 beschrieben ein mikroporöses, opakes PUR-Material mit einer Shore Härte A von 80 bis 85. Auch diese Reaktionsmischung ist bei Raumtemperatur lagerstabil; in der Hitze (120 bis 140°) erfolgt die Verfestigung innerhalb von wenigen Minuten.

**Beispiel 13**

100 g eines Polyolgemisches gemäß Beispiel 1 werden mit 30,7 g festem 4,4'-Diisocyanatodiphenylmethan (MDI, hergestellt durch Einlaufen einer MDI-Schmelze in Waschbenzin) vermischt.

Dieses Gemisch ist nicht lagerstabil, es erfolgt bereits nach wenigen Minuten starker Viskositätsanstieg (Verarbeitungszeit: 5-7 Minuten bei RT).

Der Probekörper wird noch 1 Stunde bei 120° getempert. Nach dieser Zeit erhält man ein transparentes, weiches PUR-Material Mit einer Shore Härte A von 65-70.

**Beispiel 14**

100 g eines Polyolgemisches gemäß Beispiel 4 werden bei Raumtemperatur mit 24,8 g NDI gemäß Beispiel 11) vermischt. Dieses Gemisch ist bis zu einem Temperaturbereich von 60 bis 80°C viskositätsstabil. Nach kurzem Entgasen im Wasserstrahlvakuum wird das Gemisch in eine Form gegossen, die dann auf 120 bis 140°C erhitzt wird. Es tritt rasch Verfestigung ein und man erhält einen Polyurethankunststoff mit den Werten:
Shore Härte A: 85
Stoßelastizität: 40 % (DIN 53 512).

**Patentansprüche**

1. Verfahren zur Herstellung von aliphatisch gebundene Amid- bzw. Urethangruppen enthaltenden Verbindungen aus aliphatisch gebundene Ester bzw. Carbonatgruppen enthaltenden Verbindungen, dadurch gekennzeichnet, daß aliphatisch gebundene Ester- bzw. Carbonatgruppen enthaltende Verbindungen mit mindestens einer aliphatisch gebundene Amingruppe enthaltenden Verbindungen in Gegenwart von katalytisch wirksamen Mengen an Alkalialkylaten und Alkylformiaten umgesetzt werden.

2. Verfahren nach Anspruch 1, dadurch gekennzeichnet, daß als Alkalialkylate Methylate und/oder Ethylate des Lithiums, Natriums oder Kaliums, bevorzugt Natriummethylat, eingesetzt werden.

3. Verfahren nach Anspruch 1 oder 2, dadurch gekennzeichnet, daß als Alkylformiate Methylformiat und/oder Ethylformiat, bevorzugt Methylformiat, eingesetzt werden.

4. Verfahren nach einem der Ansprüche 1 bis 3, dadurch gekennzeichnet, daß als aliphatisch gebundene Carbonatgruppen enthaltende Verbindungen Hydroxylgruppen enthaltende aliphatische Polycarbonate eingesetzt werden.

5. Verfahren nach einem der Ansprüche 1 bis 3, dadurch gekennzeichnet, daß als aliphatisch gebundene Estergruppen enthaltende Verbindungen Hydroxylgrüppen enthaltende aliphatische Polyester eingesetzt werden.

6. Verfahren nach einem der Ansprüche 1 bis 5, dadurch gekennzeichnet, daß als mindestens eine aliphatisch gebundene Amingruppe enthaltende Verbindung ein aliphatisches Polyamin eingesetzt wird.

7. Verfahren nach einem der Ansprüche 1 bis 5, dadurch gekennzeichnet, daß die Umsetzung nach Bildung mindestens einer Amid- bzw. Urethangruppe durch Säurezusatz gestoppt wird.

8. Verfahren nach Anspruch 6, dadurch gekennzeichnet, daß als Säure eine anorganische oder organische Säure mit einem pKa-Wert von Kleiner als 3 eingesetzt wird.

**9.** Verfahren nach Ansprüchen 4, 5 und 6 zur Herstellung eines Polyolgemisches mit freien Amino-Endgruppen, dadurch gekennzeichnet, daß ein Amin mit mindestens 2 endständigen aliphatisch gebundenen Aminogruppen und ein aliphatisches Polycarbonat bzw. ein aliphatischer Polyester mit mindestens 2 endständigen Hydroxylgruppen umgesetzt werden und die Umsetzung gestoppt wird, wenn mindestens eine der Aminogruppen (pro Molekül) zur Urethangruppe bzw. zur Amidgruppe umgesetzt ist.

**10.** Verwendung des Verfahrensproduktes nach Anspruch 9 als Kettenverlängerungsmittel bei der Herstellung von Polyurethankunststoffen nach dem Isocyanat-Polyadditionsverfahren.

Europäisches Patentamt

# EUROPÄISCHER RECHERCHENBERICHT

Nummer der Anmeldung
EP 94 10 6666

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A | DE-A-38 35 741 (LENTIA GMBH)<br>* Ansprüche 1,3 *<br>--- | 1,2 | C08G63/91<br>C08G64/42<br>C07C231/02<br>C07C269/04 |
| A | DATABASE WPI<br>Week 9240,<br>Derwent Publications Ltd., London, GB;<br>AN 92-328114<br>& JP-A-4 235 954 (MITSUBISHI GAS CHEM CO INC) 25. August 1992<br>* Zusammenfassung *<br>----- | 1,3,6 | |
| | | | **RECHERCHIERTE SACHGEBIETE (Int.Cl.5)** |
| | | | C07C<br>C08G |

Der vorliegende Recherchenbericht wurde für alle Patentansprüche erstellt

| Recherchenort | Abschlußdatum der Recherche | Prüfer |
|---|---|---|
| BERLIN | 23. August 1994 | Angiolini, D |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)